# EUROPEAN PATENT SPECIFICATION

(11) **EP 3 839 058 B1**
(45) Date of publication and mention of the grant of the patent: **17.05.2023**
(21) Application number: 19217885.3
(22) Date of filing: 19.12.2019
(51) Int. Cl.: C12Q 1/24

(54) **A MULTIPLE-STAGE METHOD FOR THE PROCESSING OF AIRBORNE CYANOBACTERIA IN AIR-WATER INTERFACE EMISSIONS**
MEHRSTUFIGES VERFAHREN ZUR VERARBEITUNG VON CYANOBAKTERIEN IN LUFT-WASSER-SCHNITTSTELLEN
PROCÉDÉ À PLUSIEURS ÉTAPES POUR LE TRAITEMENT DE CYANOBACTERIES AÉRIENNES DANS DES ÉMISSIONS D'INTERFACE AIR-EAU

(30) Priority: 18.12.2019 PT 2019115995
(43) Date of publication of application: 23.06.2021
(73) Proprietor: Universidade de Évora, 7000-803 Évora (PT)
(72) Inventor: MIGUEL ANTUNES, CÉLIA MARIA, 7000-671 ÉVORA (PT); BUGALHO OLIVEIRA RODRIGUES COSTA, ANA CRISTINA, 7000-671 ÉVORA (PT); MARCHÃ PENHA, MARIA ALEXANDRA, 7005-345 ÉVORA (PT); QUEIROZ MARTINS MANTERO MORAIS, MARIA MANUELA, 7005-345 ÉVORA (PT); BATISTA DA COSTA GUERREIRO DE NOVAIS, MARIA HELENA, 7005-345 ÉVORA (PT); MORALES LUIZAGA, EDUARDO ANTONIO, BOLIVIA (BO)
(74) Representative: Neves, Ana

(56) References cited:
- MURBY A L ET AL: "Field and laboratory methods to monitor lake aerosols for cyanobacteria and microcystins", AEROBIOLOGIA, SPRINGER-VERLAG, DORDRECHT, NL, vol. 32, no. 3, 3 November 2015 (2015-11-03), pages 395-403, XP036038021, ISSN: 0393-5965, DOI: 10.1007/S10453-015-9409-Z [retrieved on 2015-11-03]
- S. A. WOOD ET AL: "Quantitative assessment of aerosolized cyanobacterial toxins at two New Zealand lakes", JOURNAL OF ENVIRONMENTAL MONITORING, vol. 13, no. 6, 1 January 2011 (2011-01-01), page 1617, XP55662473, GB ISSN: 1464-0325, DOI: 10.1039/c1em10102a
- HAIG C W ET AL: "Bioaerosol sampling: sampling mechanisms, bioefficiency and field studies", JOURNAL OF HOSPITAL INFECTION, ELSEVIER, AMSTERDAM, NL, vol. 93, no. 3, 1 April 2016 (2016-04-01), pages 242-255, XP029597276, ISSN: 0195-6701, DOI: 10.1016/J.JHIN.2016.03.017
- SAVVAS GENITSARIS ET AL: "Airborne Algae and Cyanobacteria: Occurrence and Related Health Effects", FRONTIERS IN BIOSCIENCE, vol. 3, 1 January 2011 (2011-01-01), pages 772-787, XP55662535,

## Description

### TECHNICAL FIELD

The present disclosure relates to a multiple-stage method for the processing of airborne Cyanobacteria and Cyanotoxins present in air-water interface emissions, for environmental air quality monitoring purposes.

### BACKGROUND

Cyanobacteria and their toxins were already identified in aerosols, with harmful consequences to human and animal health. This algal group, as other algal groups, are naturally present in fresh, marine and brackish waters. However, they can reach very high densities (called blooms) when resulting from human activities that enrich the water with nitrogen and phosphorus (WHO, 2015).

These blooms can occur in nearly all parts of the world and can be favoured by environmental conditions, namely the high concentration of nutrients especially phosphorus (>25-50 µg L⁻¹), high water temperature (>25°C), long hydraulic retention time (>1 month) and stable water body stratification (WHO, 2015).

Climate change (by impact on water temperature and precipitation), together with eutrophication, can increase the frequency, duration and intensity of Cyanobacterial blooms (WHO, 2015).

Some features of Cyanobacteria give them advantages in the competition with other microorganisms, namely their capacity to control the buoyancy and seek water depths with optimal growth conditions (WHO, 2015), or their ability to fix atmospheric nitrogen (Whitton & Potts, 2000).

Cyanobacterial blooms affect ecosystems and their biota, including human beings, by contributing to aesthetic problems, impairing recreational use (due to surface scums and unpleasant odours), affecting the taste of drinking water, and especially by the health threats posed by Cyanotoxins, the bioactive substances of varying chemical nature secreted by Cyanobacteria

The worldwide reports of massive fish and avian kills, as well as carcinogenic effects and even human deaths are becoming more frequent, but relatively little effort is being invested in finding real solutions, all these as ever increasing rates of contamination and climate change affect ecosystems around the world.

Furthermore, the risk is not only for organisms living in water, but also for those in the proximities of affected waterbodies. These toxins can be produced by most of surface bloom-forming Cyanobacterial species, with different Cyanotoxins being potentially produced by different genera (WHO, 2015), and up to 75% of blooms being toxic (Chen, Burke & Prepas, 2011).

The mechanisms of this toxicity are very diverse and range from hepatotoxic, neurotoxic and dermatotoxic effects to general inhibition of protein synthesis (Sivonen & Jones, 1999).

The first algal sampling procedure dates back to 1935, called "the Sky Hook" designed by Colonel Lindbergh with specific requirements, such as light in weight and simple to operate. Although there were some difficulties concerning the collecting medium containers, in order to prevent contamination, they should be sufficiently strong to stand possible rough handling without breakage. This device was used on the top of an airplane (Meier, 1935).

The most widely used methods of sampling airborne algae and Cyanobacteria are using plates with selective culture media where microorganisms are cultured after passive transfer or active impact. Using these techniques, only the viable fraction of airborne Cyanobacteria is sampled, disregarding the non-viable fraction that can still cause allergic reactions and that are also transferred through air (Genitsaris et al., 2011).

In recent experiments, Cyanobacteria and their toxins were separated from aerosols formed in enclosed in vitro and field experiments (Murby & Haney, 2016; May et al., 2018).

Bioaerosols containing Cyanobacterial cells and their toxins were rarely studied before. The existing studies are reported to enclosed situations in the laboratory and in the field, using techniques that are not easy to implement, and that only collect very small volumes of air, making the study of cells and chemicals a difficult task.

Several methods were already described for bioaerosol sampling, based on passive or active systems (Haig et al., 2016).

However, despite these recent advances, little is known on the aerial transport of these organisms and their toxins, namely the mode of Cyano-aerosol formation and the development of effective ways to sample large volumes in the field needs further study.

The major limitations of these Cyanobacterial sampling technologies are that they are based on a passive gravimetric harvesting or slow flow rate, as well as they are not easy to implement in different environmental settings and collect very small volumes of air. Therefore, due to the difficulty to collect a sample, no significant amount of sample required for an accurate quantification is harvested, so that it can represent the true amount in the air, and making the visualization of cells and measurement of chemicals in suspension a difficult task. Furthermore, these procedures are time-consuming, for instance, the air kit device presented in Murby & Haney (2016) pumped the air at a flow rate of 2 L/min and the field sampling lasted 4 h.

Also, the fact that those sampling procedures are based on passive sampling is dependent on the weather conditions at each sampling site, namely the wind speed and direction. In addition to these features, all the methods disclosed in the state of the art also present disadvantages, such as: they are not easy to implement in different environmental settings; they are not repeatable in different locations since they depend on wind conditions; they do not represent accurately Cyanobacteria concentrations in the air; and they are time-consuming since they take long periods to collect sufficient material for subsequent analysis.

Thus, there is a need to adopt a sampling procedure that is independent of weather conditions and that could be repeatable in different locations.

Although the state of the art shows the air-sampler µ Coriolis, Bertin Technologies (US 2016/0238493 A1), a portable device developed for the collection of air particles and microorganisms, the referred device is not established to collect Cyanobacteria nor Cyanotoxins.

These facts are disclosed in order to illustrate the technical problem addressed by the present disclosure.

### GENERAL DESCRIPTION

The present disclosure relates to a multiple-stage method for the collection and processing of airborne Cyanobacteria and Cyanotoxins, present in air-water interface emissions, for environmental air quality monitoring purposes.

The referred process comprises multi-analytical procedures for the detection and quantification of Cyanobacteria and Cyanotoxins, allowing the evaluation of the exposure risk for humans and other animals.

The present disclosure relates to a simple, fast, and not expensive process, that allows a more efficient and accurate sampling of the airborne Cyanobacteria and Cyanotoxin, contributing to enhanced environmental air quality monitoring protocols.

The invention in its general sense is a method defined according to independent claim 1.

The present disclosure relates to a processing method wherein the collected airborne Cyanobacteria sample is processed under aseptic conditions.

The multiple-stage process allows the detection and quantification of airborne Cyanobacteria and Cyanotoxins, collected from an air-sampler provided with empty sterile cones. The air-sampler is positioned at the air-water interface at a height over water in the range between 30 and 250 cm, preferably between 70 and 200 cm over water surface. For the collection of the airborne Cyanobacteria sample, the air sampler is switched on to aspirate, using a flow rate in a range of 100 L/min to 300 L/min of air for periods between 10 to 60 minutes.

The flow rate is greater than 100 L/min, preferably 150 to 200 L/min. The sampling duration occurs for a period of 10, 20 and/or 30 minutes, preferably 30 minutes.

After collection the cones are closed and stored refrigerated under a temperature range of 2 to 8 °C for up to 2 days. After storage, the collected samples can be resuspended by rinsing the cones with a sterile liquid, selected from sterilized ultra-pure water, a saline solution or another suitable buffer, preferably up to 3 mL of sterilized ultra-pure water. The resuspended sample is then homogenised by means of vortex.

In an embodiment, a saline solution may be a suitable buffer.

In an embodiment, multi analytical procedures can be performed from the resuspended solution, including Cyanobacteria culture, Cyanotoxin screening, and microscopy analysis.

In an embodiment, 0.5 mL of the resuspended solution can be inoculated in appropriate culture media, such as BG-11, to prepare a Cyanobacteria culture.

In an embodiment, part of the resuspended solution, preferably 2/3 of the resuspended solution, can be transferred to a microtube and subsequently centrifuged for the sedimentation of Cyanobacteria.

In an embodiment, the centrifugation is performed between 5,000 and 15,000 g, preferably 9,000 to 11,000 g; and for 5 to 25 minutes, preferably 10 to 20 minutes.

In an aspect of the present disclosure, the supernatant can be stored for free Cyanotoxin screening. The pellet can be concentrated by resuspending in an appropriate volume of sterile ultra-pure water, preferably 100 µL of sterile ultra-pure water.

In an embodiment, slides for microscopy analysis can be prepared with an appropriate volume of the concentrated suspension, preferably 5 to 10 µL. Slides are prepared by using conventional mounting media for optical and/or fluorescence microscopy analysis for Cyanobacteria identification and quantification.

### BRIEF DESCRIPTION OF THE DRAWINGS

The following figures provide preferred embodiments for illustrating the disclosure and should not be seen as limiting the scope of invention.

The present disclosure is best understood from the following detailed description when read in connection with the accompanying drawing. It is emphasized that, according to common practice, the various features of the drawing are not to scale. On the contrary, the dimensions of the various features are arbitrarily expanded or reduced for clarity.

Included in the drawing are the following figures:

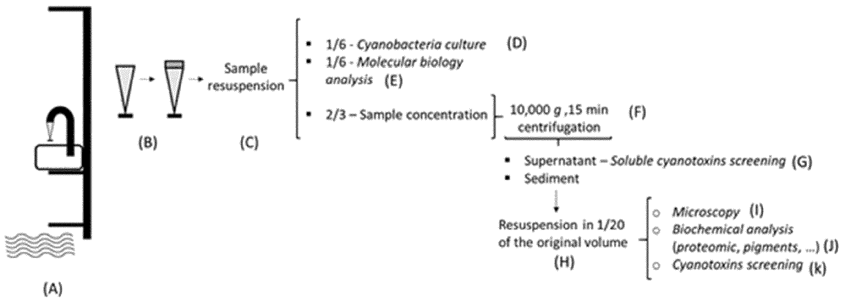

**Figure 1****:** Schematic representation of an embodiment of the methodological procedure. (A) Collection of samples using µ Coriolis, in sterilized cones and at a flow rate of 200 L/min, at desired heights over the water surface; (B) After collection, the cones are closed with screw cap and stored in refrigerated conditions (2-8°C) for up to 2 days; (C) Samples are processed under aseptic conditions, rinsing the cones with an appropriate sterile liquid; (D) For cyanobacteria cultures, 1/6 of the freshly obtained suspension is inoculated in specific culture media; (E) For molecular biology analysis, 1/6 of the suspension can be either used fresh, or deep-frozen until analysis; (F) The remaining volume is subsequently centrifuged at 10,000 g for 15 min for the sedimentation of cyanobacteria; (G) The supernatant can be stored for free Cyanotoxin screening; (H) The pellet can be concentrated by resuspending in 1/20 of the original volume, allowing other applications: (I) microscopy analysis; (J) Biochemical analysis, as chlorophyll and phycobiliproteins quantification; (K) Cyanotoxin screening.

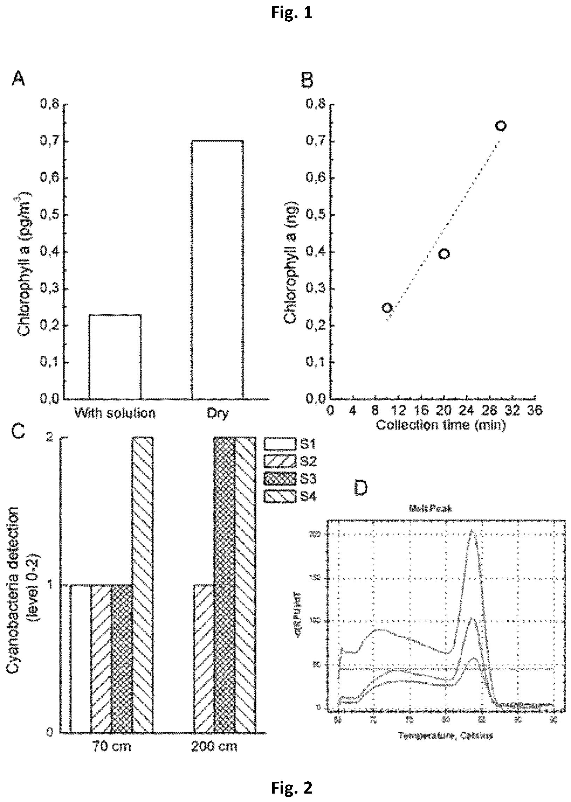

**Figure 2****:** Illustration of the results obtained from a preferred embodiment of the present disclosure. Practical applications of the invention. (A) Advantage of the dry sample, evidenced by the Chlorophyll *a* detected in the samples (ng/m³ of air); (B) Linear relation between collection time and the chlorophyll *a* detected in the samples; (C) Effect of the height of collection (70 and 200 cm over water) in the level of Cyanobacterial DNA detected (0 - not detected; 1 - intermediate level; 2 - maximal level), in four sites (S1-S4) of the same reservoir; (D) Example of a typical real time Polymerase Chain Reaction run showing melting curve analysis for Cyanobacterial detection (16S amplification).

### DETAILED DESCRIPTION

The present invention again relates in its general sense to a method defined according to independent claim 1.

The present disclosure relates to a multiple-stage method for the processing of airborne Cyanobacteria and Cyanotoxins present in air-water interface emissions, for environmental air quality monitoring purposes.

The process starts by collecting the airborne Cyanobacteria and Cyanotoxin samples at the air-water interface, using the air-sampler µ Coriolis, from Bertin Technologies. The equipment is positioned at the air-water interface, as showed in Figure 1A, at an appropriate height over water, preferably within a range between 30 and 250 cm.

The air aspiration flow rate should be sufficiently high to allow the collection of reliable and representative samples in few minutes, thus contributing to a more efficient field sampling. The collection should take periods between 10 to 60 minutes, preferably 30 minutes.

During outdoor sampling the autonomy of the µ Coriolis equipment (1h) also needs to be considered. For the purpose of the present invention, the air flow rate is in a range of 100 L/min to 300 L/min. For one embodiment of the present invention, the optimum flow rate is 200 L/min, since it allows faster sampling than at 100 L/min, and the equipment revealed some instability with flow rates of 300 L/min (the maximum flow rate referred in the equipment specifications).

The air sampler equipment should be equipped with empty sterile cones, as the use of a liquid in the cones reduces the amount of Cyanobacteria that can be collected, see Figure 2A. After collection, the cones are closed as showed in Figure 1B, with the respective screw lid, and stored refrigerated under a temperature range of 2 to 8 °C for up to 2 days.

The samples can be resuspended and recovered after the collection and refrigeration, by rinsing the cones with an appropriate sterile liquid, preferably sterilized ultra-pure water (SUW). Other specific buffers, including a saline solution can also be used, considering the subsequent analytical procedures (Figure 1C) such as culture procedures, molecular biology analysis or, after concentration, biochemical characterization and several microscopy analyses.

An air sample was collected with the equipment positioned 70 and 200 cm over water, as showed on Figure 2C, using an aspiration flow rate of 200 L/min. Sampling duration was 10, 20 and 30 minutes. After the sampling process, collection and refrigeration, samples were processed under aseptic conditions in a biological safety class 2 flow chamber and using aseptic techniques. The resuspension of the samples was performed by addition of up to 3 mL of sterilized ultra-pure water (SUW) in the cone and vortex to improve the suspension homogenising for up to 2 minutes to ensure total homogenisation. The obtained suspension can be separated into various aliquots for subsequent analysis.

An aspect of the disclosure comprises a preparation of Cyanobacteria culture, wherein specific culture media, such as BG-11, is inoculated with 0.5 mL of the freshly obtained suspension, as showed in the Figure 1D. Another aspect of the present disclosure relates to the preparation of the sample for molecular biology analysis, wherein 0.5 mL (or 1/6) of the collected suspension can be either freshly used or deep-frozen (-80 °C) until analysis (Fig. 1E). An embodiment of Cyanobacterial DNA detection is shown in figures 2C and 2D. The reminiscent volume (2/3) can be transferred to a microtube and subsequently centrifuged at 10,000 g for 15 minutes for the sedimentation of Cyanobacteria (Fig. 1F). The supernatant can be stored for free Cyanotoxin screening (Fig. 1G) and the pellet can be concentrated by resuspending in 100 µL of SUW (Fig. 1H).

Figure 2B shows the results obtained from the previous embodiment. The mesocosms experiments revealed that with a sampling duration of 30 minutes, the concentration of Cyanobacteria was higher than in samples collected at 10 or 20 minutes. This sampling duration, such as 30 minutes, also allows the collection of 2 samples using the equipment own battery, contributing also to the efficiency in outdoor sampling.

In an embodiment, slides are prepared with 5-10 µL of the concentrated suspension and using conventional mounting media for optical and fluorescence microscopy analysis for Cyanobacteria identification and quantification (Fig. 1I).

In an embodiment, the concentrated suspension may also be analysed by atomic force or scanning electronic microscopy for structure and elemental analysis.

The reminiscent suspension can be deep-frozen and stored until biochemical characterisation, as it is appropriate for evaluation of the total protein content (Fig. 1J), and/or of the photosynthetic pigments (chlorophyll and phycobiliproteins, for example), and Cyanotoxin screening (Fig. 1K).

### References:

Chen H., Burke E. & Prepas E.E. (2011). Cyanobacterial toxins in fresh waters. In: Nriagu J.O. (Ed.), Encyclopedia of environmental health. Amsterdam: Elsevier; p. 860-871.
Genitsaris S., Kormas K. & Moustaka-Gouni M. (2011). Airborne algae and Cyanobacteria: Occurrence and related health effects. Frontiers in bioscience 3: 772-87.
Haig C.W., Mackay W.G., Walker J.T. & Williams C. (2016). Bioaerosol sampling: sampling mechanisms, bioefficiency and field studies. Journal of Hospital Infection, 93: 242-255. https://doi.org/10.1016/i.jhin.2016.03.017
May N.W., Olson N.E., Panas M., Axson J.L., Tirella P.S., Kirpes R.M., Craig R.L., Gunsch M.J., China S., Laskin A., Ault A.P. & Pratt K.A. (2018). Aerosol emissions from Great Lakes Harmful Algal Blooms. Env. Sci. Technol. 52: 397-405.
Meier F.C. & Lindbergh C.A. (1935). Collecting Micro-Organisms from the Arctic Atmosphere: With Field Notes and Material. The Scientific Monthly 40: 5-20. www.jstor.org/stable/15761.
Murby A.L. & Haney J.F. (2016). Field and laboratory methods to monitor lake aerosols for Cyanobacteria and microcystins. Aerobiol. 32: 395-403.
Sivonen K. & Jones G. (1999). Cyanobacterial Toxins. In: Chorus I. & Bartram J. (Eds), Toxic Cyanobacteria in Water: A Guide to Their Public Health Consequences, Monitoring, and Management, pp. 41-111. London.
Whitton B.A. & Potts M. (2000). Introduction to the Cyanobacteria. In: Whitton B.A. & Potts M. (Eds),. The Ecology of Cyanobacteria, pp. 1-11. Kluwer Academic Publishers, Netherlands.
World Health Organization (2015) Management of Cyanobacteria in drinking-water supplies: Information for regulators and water suppliers, 11pp. WHO/FWC/WSH/15.03

## Claims

1. A multiple-stage method for the processing of airborne Cyanobacteria and Cyanotoxins in air-water interface emissions, comprising the following steps:
collecting an airborne Cyanobacteria or Cyanotoxins sample at the air-water interface using an air-sampler which is a portable device including a cyclone enclosure for centrifuging air, external air inlet for admitting air into the enclosure, and air outlet connected by a coupling to an air inlet filter of an individual motorized respiratory protection appliance, provided with empty sterile cones and wherein:
- the air-sampler is positioned at the air-water interface at a height over water in the range between 30 and 250 cm, preferably between 70 and 200 cm over water surface;
- and the air-sampler is switched on to aspirate and a flow rate is in a range of 100 L/min to 300L/min of air for periods between 10 to 60 minutes, preferably 100 to 200 L/min with a sampling duration of 10, 20 and/or 30 minutes, preferably 30 minutes;
storing airborne Cyanobacteria sample under a temperature range of 2 to 8 °C for up to 2 days;
resuspending the airborne Cyanobacteria sample;
homogenising the airborne Cyanobacteria sample;
inoculating or deep-freeze the obtained suspension in the previous step;
centrifuging the reminiscent volume for the sedimentation of Cyanobacteria;
storing the supernatant and concentring the pellet by resuspending;
preparing a slide for microscopy identification;
further comprising the Cyanotoxin quantification from the supernatant;

2. The multiple-stage process according to the previous claim, wherein the cones are closed and stored refrigerated under a temperature range of 2 to 8 °C for up to 2 days.

3. The multiple-stage process according to any of the previous claims, wherein the samples are resuspended by rinsing the cones with a sterile liquid for homogenization, selected from sterilized ultra-pure water, or a suitable buffer, preferably 3 mL of sterilized ultra-pure water.

4. The multiple-stage process according to any of the previous claims, wherein the resuspended sample is used to perform analytical assays, including multiple analytical assays with the same sample.

5. The multiple-stage process according to any of the previous claims, wherein a Cyanobacteria culture is prepared by inoculating a suitable culture media with the freshly obtained suspension, preferably with 0.5 mL of the freshly obtained suspension.

6. The multiple-stage process according to any of the previous claims, wherein a part of the resuspended solution is transferred to a microtube and subsequently centrifuged for the sedimentation of Cyanobacteria, preferably 2/3 of the resuspended solution.

7. The multiple-stage process according to the previous claim, wherein the centrifugation is performed:
between 5,000 and 15,000 g, preferably 9,000 to 11,000 g; and for 5 to 25 minutes, preferably 10 to 20 minutes.

8. The multiple-stage process according to any of the previous claims, wherein the supernatant is stored for free Cyanotoxin screening and the pellet is concentrated by resuspending in an appropriate volume of sterile ultra-pure water, preferably 100 µL of sterile ultra-pure water.

9. The multiple-stage process according to any of the previous claims, wherein slides for microscopy analysis are prepared with an appropriate volume of the concentrated suspension, preferably 5 to 10 µL.

10. The multiple-stage process according to the previous claim, wherein the slides are prepared by using conventional mounting media for optical and/or fluorescence microscopy analysis for Cyanobacteria identification and quantification.

## Patentansprüche

1. Eine mehrstufige Methode für die Verarbeitung von luftgetragenen Cyanobakterien und Cyanotoxinen in Luft-Wasser-Grenzflächenemissionen, die aus folgenden Schritte besteht:
Entnahme einer luftgetragenen Cyanobakterien- oder Cyanotoxinprobe an der Luft-Wasser-Grenzfläche unter Verwendung eines Luftkeimsammlers, der aus einem tragbaren Gerät mit einem Zyklongehäuse zum Zentrifugieren der Luft, einem externen Lufteinlass für den Eintritt der Luft in das Gehäuse sowie aus einem Luftauslass besteht, der durch eine Kupplung mit einem Lufteinlassfilter eines einzelnen motorisierten Atemschutzgeräts verbunden ist, das mit leeren sterilen Konen versehen ist und worin:
- Der Luftkeimsammler an der Luft-Wasser-Grenzfläche in einer Höhe im Bereich zwischen 30 und 250 cm über dem Wasser, vorzugsweise zwischen 70 und 200 cm über der Wasseroberfläche, positioniert wird;
- und der Luftkeimsammler zum Ansaugen eingeschaltet wird und eine Durchflussmenge im Bereich von 100 l/min. bis 300 l/min. Luft für Zeiträume zwischen 10 bis 60 Minuten aufweist, die vorzugsweise 100 bis 200 l/min. bei einer Probenahmedauer von 10, 20 und/oder 30 Minuten, vorzugsweise 30 Minuten, beträgt;
Lagerung der luftgetragenen Cyanobakterienprobe bei einem Temperaturbereich von 2 °C bis 8 °C für bis zu 2 Tage;
Resuspendieren der luftgetragenen Cyanobakterienprobe;
Homogenisieren der luftgetragenen Cyanobakterienprobe;
Impfen oder Tiefkühlen der im vorherigen Schritt erhaltenen Suspension;
Zentrifugieren des erinnernden Volumens für die Sedimentation der Cyanobakterien;
Lagerung des Überstands und Konzentration des Pellets durch Resuspendieren;
Vorbereitung eines Objektträgers für die mikroskopische Identifikation;
zusätzlich bestehend aus der Cyanotoxin-Quantifizierung aus dem Überstand.

2. Das mehrstufige Verfahren gemäß dem vorhergehenden Anspruch, worin die Konen geschlossen und bei einem Temperaturbereich von 2 bis 8 °C bis zu 2 Tage gekühlt gelagert werden.

3. Das mehrstufiges Verfahren gemäß einem der vorhergehenden Ansprüche, worin die Proben durch Spülen der Konen mit einer sterilen Flüssigkeit zur Homogenisierung resuspendiert werden, die aus sterilisiertem Reinstwasser, oder einem geeigneten Puffer, vorzugsweise 3 ml sterilisiertem Reinstwasser ausgewählt wird.

4. Das mehrstufiges Verfahren gemäß einem der vorhergehenden Ansprüche, worin die resuspendierte Probe zur Durchführung analytischer Untersuchungen, einschließlich von mehrfachen analytischen Untersuchungen mit derselben Probe verwendet wird.

5. Das mehrstufiges Verfahren gemäß einem der vorhergehenden Ansprüche, worin eine Cyanobakterienkultur hergestellt wird, indem ein geeignetes Kulturmedium mit der frisch erhaltenen Suspension, vorzugsweise mit 0,5 ml der frisch erhaltenen Suspension, geimpft wird.

6. Das mehrstufiges Verfahren gemäß einem der vorhergehenden Ansprüche, worin ein Teil der resuspendierten Lösung in ein Mikroröhrchen überführt und anschließend zur Sedimentation von Cyanobakterien zentrifugiert wird, wobei vorzugsweise 2/3 der resuspendierten Lösung überführt werden.

7. Das mehrstufiges Verfahren gemäß dem vorhergehenden Anspruch, worin die Zentrifugation folgendermaßen durchgeführt wird:
Zwischen 5.000 und 15.000 g, vorzugsweise 9.000 bis 11.000 g, für einen Zeitraum von 5 bis 25 Minuten, vorzugsweise von 10 bis 20 Minuten.

8. Das mehrstufige Verfahren gemäß einem der vorhergehenden Ansprüche, worin der Überstand für das Screening auf freies Cyanotoxin gelagert wird und das Pellet durch Resuspendieren in einem geeigneten Volumen sterilen Reinstwassers, vorzugsweise 100 pl sterilen Reinstwassers, konzentriert wird.

9. Das mehrstufige Verfahren gemäß einem der vorhergehenden Ansprüche, worin die Objektträger für die mikroskopische Analyse mit einem geeigneten Volumen der konzentrierten Suspension, vorzugsweise von 5 bis 10 pl, vorbereitet werden.

10. Das mehrstufige Verfahren gemäß dem vorhergehenden Anspruch, worin die Objektträger unter Verwendung herkömmlicher Fixiermittel für die optische und/oder fluoreszenzmikroskopische Analyse zur Identifizierung und Quantifizierung der Cyanobakterien hergestellt werden.

## Revendications

1. Procédé à plusieurs étapes pour le traitement de cyanobactéries et de cyanotoxines en suspension dans des émissions d'interface air-eau, comprenant les étapes suivantes :
la collecte d'un échantillon de cyanobactéries ou de cyanotoxines en suspension dans l'air à l'interface air-eau à l'aide d'un échantillonneur d'air qui est un dispositif portable comprenant une enceinte à cyclone pour la centrifugation de l'air, une entrée d'air externe pour l'admission de l'air dans l'enceinte et une sortie d'air reliée par un couplage à un filtre d'entrée d'air d'un dispositif de protection respiratoire motorisé individuel, fourni avec des cônes stériles vides et dans lequel :
- l'échantillonneur d'air est positionné à l'interface air-eau à une hauteur au-dessus de l'eau comprise entre 30 et 250 cm, de préférence entre 70 et 200 cm au-dessus de la surface de l'eau ;
- et l'échantillonneur d'air est allumé pour aspirer et le taux d'écoulement est dans une plage allant de 100 L/min à 300 L/min d'air pendant des périodes comprises entre 10 et 60 minutes, de préférence de 100 à 200 L/min avec une durée d'échantillonnage de 10, 20 et/ou 30 minutes, de préférence 30 minutes ;
le stockage de l'échantillon de cyanobactéries à une plage de températures allant de 2 à 8 °C pendant jusqu'à 2 jours ;
la remise en suspension de l'échantillon de cyanobactéries en suspension dans l'air ;
l'homogénéisation de l'échantillon de cyanobactéries en suspension dans l'air ;
l'inoculation ou la congélation de la suspension obtenue à l'étape précédente ;
la centrifugation du volume restant pour la sédimentation des cynobactéries ;
le stockage du surnageant et la concentration du sédiment par la remise en suspension ;
la préparation d'une lame pour une identification par microscope ;
comprenant en outre la quantification de cyanotoxines provenant du surnageant.

2. Procédé à plusieurs étapes selon la revendication précédente, dans lequel les cônes sont fermés et stockés à une plage de températures allant de 2 à 8 °C pendant jusqu'à 2 jours.

3. Procédé à plusieurs étapes selon l'une quelconque des revendications précédentes, dans lequel les échantillons sont remis en suspension par le rinçage des cônes avec un liquide stérile pour l'homogénéisation, sélectionné parmi de l'eau ultrapure stérilisée, ou un tampon approprié, de préférence 3 mL d'eau ultrapure stérilisée.

4. Procédé à plusieurs étapes selon l'une quelconque des revendications précédentes, dans lequel l'échantillon remis en suspension est utilisé pour effectuer des essais analytiques, comprenant plusieurs essais analytiques avec le même échantillon.

5. Procédé à plusieurs étapes selon l'une quelconque des revendications précédentes, dans lequel une culture de cyanobactéries est préparée par l'inoculation d'un milieu de culture approprié avec la suspension fraîchement obtenue, de préférence avec 0,5 mL de la suspension fraîchement obtenue.

6. Procédé à plusieurs étapes selon l'une quelconque des revendications précédentes, dans lequel une partie de la solution remise en suspension est transférée dans un microtube et ensuite centrifugée pour la sédimentation des cyanobactéries, de préférence 2/3 de la solution remise en suspension.

7. Procédé à plusieurs étapes selon la revendication précédente, dans lequel la centrifugation est effectuée : entre 5000 et 15 000 g, de préférence 9000 à 11 000 *g* ; et pendant 5 à 25 minutes, de préférence 10 à 20 minutes.

8. Procédé à plusieurs étapes selon l'une quelconque des revendications précédentes, dans lequel le surnageant est stocké pour le dépistage de cyanotoxines libres et le sédiment est concentré par la remise en suspension dans un volume approprié d'eau ultrapure stérile, de préférence 100 µL d'eau ultrapure stérile.

9. Procédé à plusieurs étapes selon l'une quelconque des revendications précédentes, dans lequel des lames pour une analyse par microscope sont préparées avec un volume approprié de la suspension concentrée, de préférence de 5 à 10 µL.

10. Procédé à plusieurs étapes selon la revendication précédente, dans lequel les lames sont préparées à l'aide d'un support de montage conventionnel pour une analyse par microscope optique et/ou à fluorescence pour l'identification et la quantification des cyanobactéries.
